# EUROPEAN PATENT APPLICATION

(11) **EP 4 756 118 A1**
(43) Date of publication of application: **10.06.2026**
(21) Application number: 24823765.3
(22) Date of filing: 17.06.2024
(51) Int. Cl.: D06H 3/08

(54) **DRAPE TESTING APPARATUS**

(30) Priority: 16.06.2023 KR 20230077228; 14.06.2024 KR 20240077666
(71) Applicant: CLO Virtual Fashion Inc., Seoul 06236 (KR)
(72) Inventor: JU, Eun Jung, Seoul 06236 (KR); KIM, Kwang Yun, Seoul 06236 (KR); YOON, Sung Jin, Seoul 06236 (KR)
(74) Representative: Gill Jennings & Every LLP
(86) International application number: PCT/KR2024/008294
(87) International publication number: WO 2024/258249

(57) **Abstract**

This drape testing apparatus comprises: a fixing body; a first moving body configured to support a center region of a fabric, to move in a direction opposite to a direction of gravity, and to be fixed to the fixing body; and a second moving body configured to support a peripheral region different from the center region of the fabric, to move in the direction opposite to the direction of gravity, and to move in the direction of gravity while the first moving body is fixed to the fixing body.

## Description

### TECHNICAL FIELD

The disclosure relates to a drape testing apparatus.

### BACKGROUND ART

Fabric drape refers to the ability of a fabric deform when hanging under its weight in a specific condition. The fabric drape depends on various properties of a fabric such as according to luster, a color, and/or texture and may define the appearance of fabric and a garment appearance. U.S. Patent No. 5,097,713 discloses an apparatus for testing the stiffness of fabrics. The above description is information the inventor(s) acquired during the course of conceiving the present disclosure, or already possessed at the time, and is not necessarily art publicly not known before the present application was filed.

### DISCLOSURE OF THE INVENTION

### TECHNICAL GOALS

An aspect of the disclosure may provide a drape testing apparatus for scanning an appearance according to a property of a fabric to collect data for estimating the property of the fabric.

### TECHNICAL SOLUTIONS

A drape testing apparatus may include a fixing body, a first moving body configured to support a center region of a fabric, to move in a direction that is opposite to a direction of gravity, and to be fixed to the fixing body, and a second moving body configured to support an adjacent region that is different from the center region of the fabric, to move in the direction that is opposite to the direction of gravity, and to move in the direction of gravity while the first moving body is fixed to the fixing body.

While the first moving body is not fixed to the fixing body, the first moving body and the second moving body may substantially simultaneously move in the direction opposite to the direction of gravity.

The second moving body may include a hole aligned with the first moving body.

The second moving body may include a protrusion configured to support the first moving body.

The drape testing apparatus may further include a fixing unit configured to magnetically fix the first moving body to the fixing body.

The drape testing apparatus may further include an actuator configured to move the second moving body in the direction opposite to the direction of gravity.

The drape testing apparatus may further include a coupler configured to couple the second moving body to the fixing body.

The fixing body may include a first leg, a second leg opposite to the first leg based on the first moving body, and an extension extending between the first leg and the second leg.

The drape testing apparatus may further include a laser emitting a laser beam towards a center of the first moving body.

The drape testing apparatus may further include a camera configured to obtain an image of the fabric and disposed below the first moving body.

The drape testing apparatus may further include a texture scanner configured to scan a texture of the fabric.

The drape testing apparatus may further include a light source at least partially illuminating the fabric.

The drape testing apparatus may further include a case configured to block light from an outside and be detachable from the fixing body.

### EFFECTS OF THE INVENTION

According to an embodiment, a consistent and natural initial drape shape of a fabric may be formed. A drape testing apparatus according to an embodiment may obtain a result of draping a fabric in a more realistic environment by a free-fall of the fabric. According to an embodiment, the drape testing apparatus may conduct a drape test in a miniaturized structure. The effects of the drape testing apparatus are not limited to the above-mentioned effects, and other unmentioned effects can be clearly understood from the above description by those having ordinary skill in the technical field to which the present disclosure pertains.

### BRIEF DESCRIPTION OF DRAWINGS

The foregoing and other aspects, features, and advantages of embodiments in the disclosure will be apparent from the following detailed description with reference to the accompanying drawings.
FIG. 1 is a perspective view of a drape testing apparatus, according to an embodiment.
FIG. 2 is a plan view of the drape testing apparatus, according to an embodiment.
FIG. 3 is a perspective view of a second moving object of the drape testing apparatus, according to an embodiment.
FIG. 4 is a side view of the drape testing apparatus, according to an embodiment.
FIG. 5 is a side view of the drape testing apparatus before the second moving object elevates, according to an embodiment.
FIG. 6 is a side view of the drape testing apparatus after the second moving object elevates, according to an embodiment.
FIG. 7 is a perspective view of the drape testing apparatus after the second moving object falls, according to an embodiment.
FIG. 8 is a perspective view of the drape testing apparatus, according to an embodiment.
FIG. 9 is an enlarged view of portion A of FIG. 4.

### BEST MODE FOR CARRYING OUT THE INVENTION

Hereinafter, embodiments will be described in detail with reference to the accompanying drawings. However, various alterations and modifications may be made to the embodiments. Here, the embodiments are not construed as limited to the disclosure. The embodiments should be understood to include all changes, equivalents, and replacements within the idea and the technical scope of the disclosure.

The terminology used herein is for the purpose of describing particular embodiments only and is not to be limiting of the embodiments. As used herein, the singular forms "a," "an," and "the" are intended to include the plural forms as well, unless the context clearly indicates otherwise. It should be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which embodiments belong. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

When describing the embodiments with reference to the accompanying drawings, like reference numerals refer to like constituent elements and a repeated description related thereto will be omitted. In the description of embodiments, detailed description of well-known related structures or functions will be omitted when it is deemed that such description will cause ambiguous interpretation of the present disclosure.

Also, in the description of the components, terms such as first, second, A, B, (a), (b) or the like may be used herein when describing components of the present disclosure. These terms are used only for the purpose of discriminating one constituent element from another constituent element, and the nature, the sequences, or the orders of the constituent elements are not limited by the terms. It should be noted that if one component is described as being "connected," "coupled" or "joined" to another component, the former may be directly "connected," "coupled," and "joined" to the latter or "connected", "coupled", and "joined" to the latter via another component.

The same name may be used to describe an element included in the embodiments described above and an element having a common function. Unless otherwise mentioned, the description of one embodiment may be applicable to other embodiments. Thus, duplicated description is omitted for conciseness.

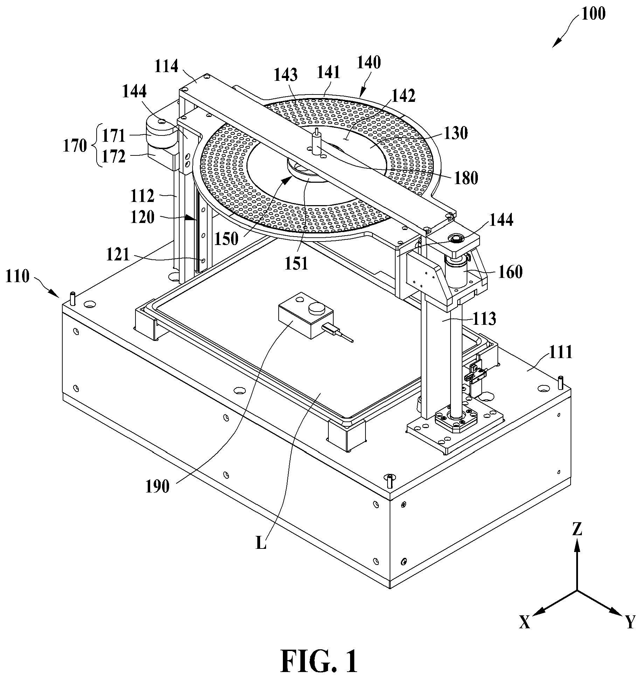

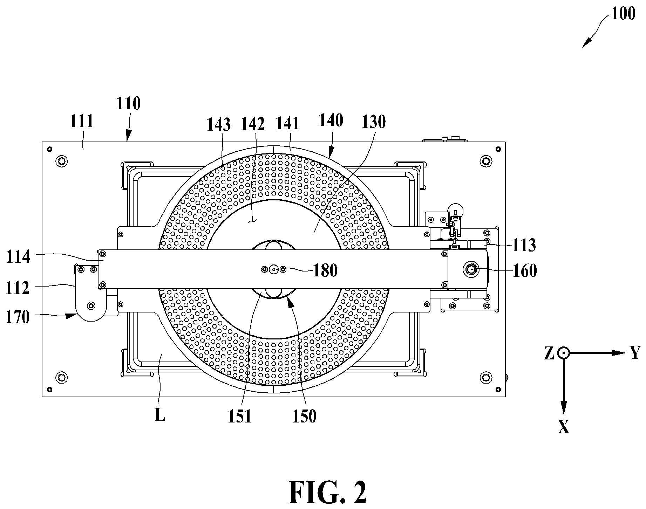

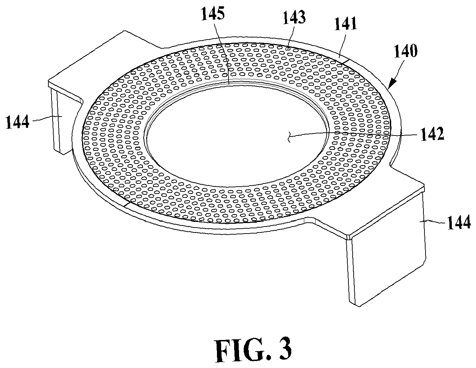

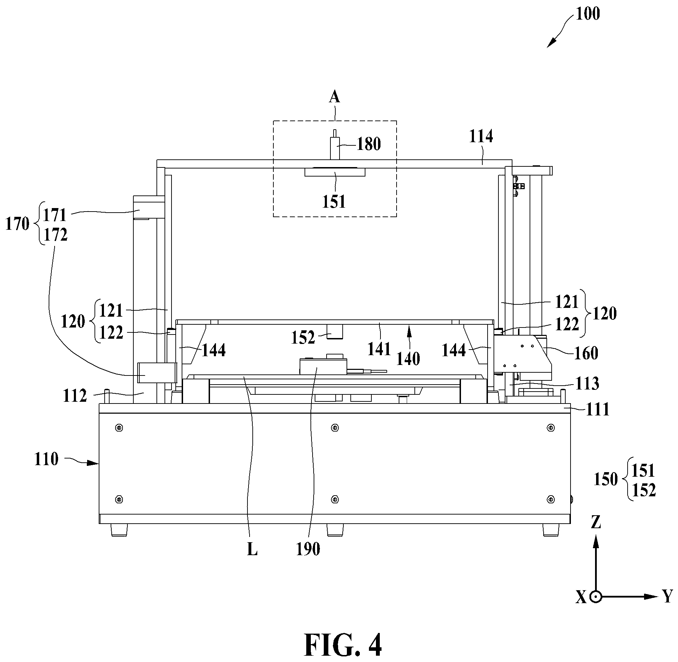

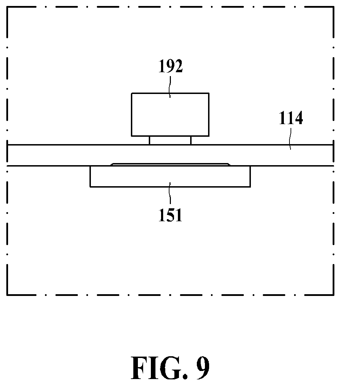

FIG. 1 is a perspective view of a drape testing apparatus according to an embodiment. FIG. 2 is a plan view of the drape testing apparatus according to an embodiment. FIG. 3 is a perspective view of a second moving object of the drape testing apparatus according to an embodiment. FIG. 4 is a side view of the drape testing apparatus according to an embodiment. FIG. 9 is an enlarged view of portion A of FIG. 4.

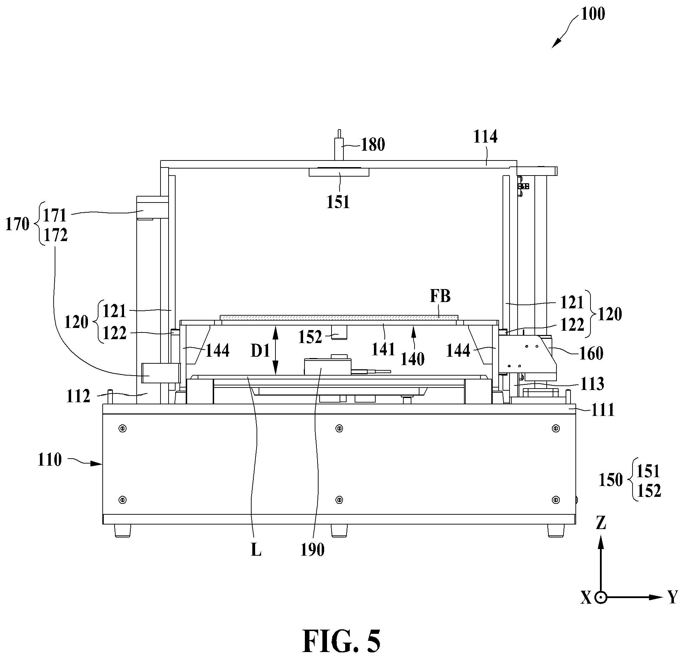

Referring to FIGS. 1 to 4, a drape testing apparatus 100 (hereinafter, also referred to as the "apparatus 100") may be used for scanning an appearance according to a property of a fabric (e.g., fabric FB of FIGS. 5 to 7) to collect data for estimating the properties of the fabric. For example, the properties of the fabric may include at least one of stretch-weft stiffness, stretch-wrap stiffness, shear stiffness, banding-weft stiffness, banding-wrap stiffness, or bending bias stiffness or a combination thereof. In this case, the weft may indicate a horizontal thread of the fabric and may also be referred to as a filling yarn. Additionally, the warp may indicate a vertical thread of the fabric and may also be referred to as a warping yarn.

The apparatus 100 may measure and/or assess an appearance change (e.g., deformation) in fabric FB due to its weight under a specific condition. The apparatus 100 may be disposed on a substantially flat surface (e.g., the ground) in an environment with the gravity is present.

The apparatus 100 may include a fixing body (a stationary structure) 110. The fixing body 110 may not substantially move relative to other components (e.g., a first moving body 130 or a second moving body 140).

The fixing body 110 may include a base 111. The base 111 may be configured to support other components (e.g., a first leg 112, a second leg 113, or a light source L). The base 111 may include an opening to which a fixing means (e.g., a screw) for fixing other components is fixed. The base 111 may be configured to provide a support surface (e.g., a surface in the +Z normal direction) on which other components are disposed. The base 111 may include an opening to which a fixing means (e.g., a screw) for fixing other components is fixed. The base 111 may include a plate shape, but is not limited thereto, and may include various shapes. The base 111 may include a metal material. For example, the base 111 may include aluminum. Other components may be disposed on the base 111.

The fixing body 110 may include a first leg 112. The first leg 112 may be disposed on the base 111. The first leg 112 may be disposed on a first portion (e.g., an edge portion in the -Y direction) of the base 111. The first leg 112 may extend from the base 111 in a normal direction (e.g., the +Z direction) of the base 111. The first leg 112 may be supported by the base 111. The first leg 112 may include a metal material. For example, the first leg 112 may include aluminum.

The fixing body 110 may include a second leg 113. The second leg 113 may be disposed on the base 111. The second leg 113 may be disposed on a position opposite to the first leg 112 based on a first moving body 130 on the base 111. The second leg 113 may be disposed on a second portion (e.g., an edge portion in the +Y direction) of the base 111. The second leg 113 may extend from the base 111 in the normal direction (e.g., the +Z direction) of the base 111. The second leg 113 may be supported by the base 111. The second leg 113 may include a metal material. For example, the second leg 113 may include aluminum.

In an embodiment that is not illustrated, the fixing body 110 may include only a single leg (e.g., the first leg 112). In an embodiment that is not illustrated, the fixing body 110 may include three or more legs (e.g., the first leg 112, the second leg 113, a third leg, and etc.) disposed on different portions of the base 111.

The fixing body 110 may include an extension 114. The extension 114 may extend between the first leg 112 and the second leg 113. The extension 114 may be connected to a first end (e.g., an end in the +Z direction) of the first leg 112. The extension 114 may be connected to a first end (e.g., an end in the +Z direction) of the second leg 113. The extension 114 may be disposed on the base 111. Other components (e.g., the first moving body 130 or the second moving body 140) may be placed between the base 111 and the extension 114.

The extension 114 may include a solid (e.g., a rectangular plate) having a polygonal cross-sectional shape. The extension 114 may be substantially flat. The extension 114 may include a metal material. For example, the extension 114 may include aluminum. The extension 114 may not substantially move in its position.

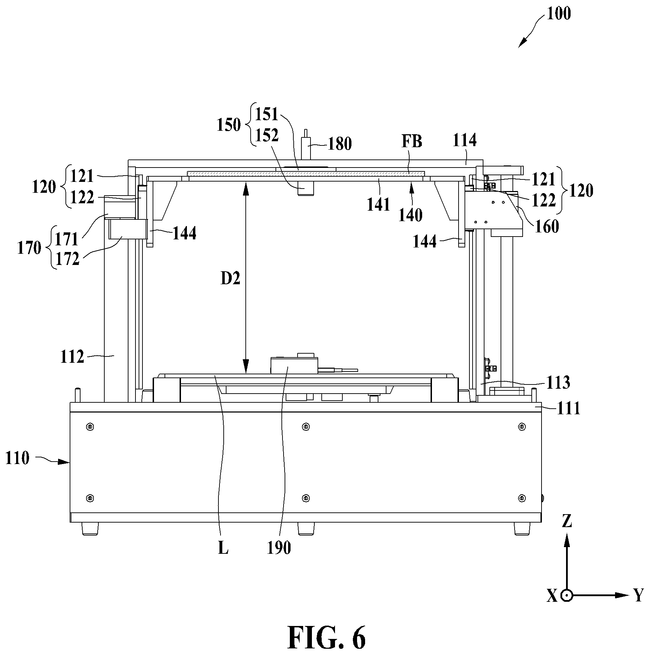

The extension114 may include the first moving body 130. The first moving body 130 may be configured to move relative to the fixing body 110. The first moving body 130 may be at least partially supported by a second moving body 140. The first moving body 130 may move (e.g., elevate) in a direction (e.g., the +Z direction) substantially opposite to the direction of the gravity. When the first moving body 130 moves in the direction (e.g., the +Z direction) substantially opposite to the direction of the gravity, the first moving body 130 may be configured to be fixed to the fixing body 110. The first moving body 130 may be configured to be fixed to the extension 114 when the first moving body 130 is moved to a predetermined position (e.g., second height D2 of FIG. 6). For example, the first moving body 130 may include a magnet. For example, when the first moving body 130 moves to a height that is substantially equal to a predetermined height (e.g., the second height D2 of FIG. 6) from the base 111, the first moving body 130 may be configured to be fixed to the extension 114 by the magnet.

The first moving body 130 may be configured to provide a support surface on which a fabric (e.g., the fabric FB of FIGS. 5 to 7) is disposed. For example, the first moving body 130 may be configured to support a region (e.g., a center region) of the fabric. For example, the support surface (e.g., a top surface) of the first moving body 130 may include a solid (e.g., a disk) having a substantially circular or elliptical cross-sectional shape. In an embodiment that is not shown, the first moving body 130 may include a solid (e.g., a rectangular plate) having a polygonal cross-sectional shape. In an embodiment that is not shown, the first moving body 130 may include a substantially spherical or hemispherical solid. The support surface of the first moving body 130 may be substantially flat. The first moving body 130 may include a metal material. For example, the first moving body 130 may include aluminum.

The apparatus 100 may include the second moving body 140. The second moving body 140 may be configured to move relative to the fixing body 110. The second moving body 140 may be configured to move (e.g., elevate) in a direction (e.g., the +Z direction) substantially opposite to the direction of the gravity. The second moving body 140 may be configured to move (e.g., fall) substantially in the direction of gravity (e.g., the -Z direction). When the first moving body 130 is fixed to the fixing body 110 or the extension 114, the second moving body 140 may be configured to move substantially in the direction of the gravity. The movement of the second moving body 140 relative to the fixing body 110 may induce natural and/or consistent appearance changes in the fabric FB.

The second moving body 140 may include a first plate 141. The first plate 141 may be configured to support a region (e.g., an adjacent region) that is different from the region (e.g., the center region) of the fabric (e.g., the fabric FB of FIGS. 5 to 7) supported by the first moving body 130. The first plate 141 may be configured to move in the direction (e.g., the +Z direction) substantially opposite to the direction of the gravity. When the first plate 141 moves in the direction (e.g., the +Z direction) substantially opposite to the direction of the gravity, the first moving body 130 may simultaneously move in the direction substantially opposite to the direction of the gravity. While the first plate 141 moves in the direction substantially opposite to the direction of the gravity, the height of a surface (e.g., a surface in the +Z normal direction) of the first moving body 130 may be substantially the same as a height of a surface (e.g., a surface in the +Z normal direction) of the first plate 141.

The first plate 141 may be configured to move (e.g., fall) substantially in the direction of the gravity (e.g., the -Z direction). When the first plate 141 moves substantially in the direction of the gravity, the first moving body 130 may be configured to be fixed to the extension 114. When the first plate 141 moves (e.g., falls) substantially in the direction of the gravity (e.g., the -Z direction), a support force of the first plate 141 on the fabric may be reduced or removed, and the region (e.g., the adjacent region) of the fabric supported by the first plate 141 may flow down in the direction of the gravity.

The first plate 141 may extend around a center axis (e.g., the Z axis) of the first plate 141. For example, the first plate 141 may include an annular shape having an inner edge and an outer edge. The center axis of the first plate 141 may substantially coincide with the center axis of the first moving body 130.

The first plate 141 may be placed outside of the first moving body 130. Before the first plate 141 moves (e.g., elevates) in the direction (e.g., the +Z direction) substantially opposite to the direction of the gravity, the position (e.g., the height from the base 111) of the first plate 141 may be substantially the same as of the position (e.g., the height from the base 111) of the first moving body 130.

The second moving body 140 may include a first hole 142. The first hole 142 may be configured to accommodate at least a portion of the first moving body 130. The first hole 142 may have a substantially circular or elliptical shape. The shape of the first hole 142 is not limited to the circular or elliptical shape and may include a shape complementary to the shape of the first moving body 130. The first hole 142 may be disposed on the center region of the first plate 141. The first hole 142 may be defined by the inner edge of the first plate 141. The first hole 142 may penetrate a first side (e.g., a side in the +Z direction) of the first plate 141 and through to a second side (e.g., a side in the -Z direction) at the opposite side of the first side. The center axis (e.g., the Z axis) of the first hole 142 may substantially coincide with the center axis (e.g., the Z axis) of the first moving body 130.

The second moving body 140 may include a plurality of second holes 143. The plurality of second holes 143 may penetrate the first side (e.g., the side in the +Z direction) of the first plate 141 through to the second side (e.g., the side in the -Z direction) at the opposite side of the first side. Air may flow through the plurality of second holes 143. For example, when the first plate 141 falls, air on the second side of the first plate 141 may flow through to the first side of the first plate 141 via the plurality of second holes 143, and thereby, air resistance of the first plate 141 may be reduced. Accordingly, the fabric may show a desired shape change (e.g., a natural and consistent shape change) when flowing down in the direction of the gravity.

The plurality of second holes 143 may include a substantially circular or elliptical cross-sectional shape. The plurality of second holes 143 is not limited to the circular or elliptical shape and may have any shape suitable for passing air. For example, the plurality of second holes 143 may include a polygonal shape, such as a rectangle or an octagon.

The plurality of second holes 143 may be arranged around the center axis of the first plate 141 and in a circumferential direction of the first plate 141. The second moving body 140 may include a plurality (e.g., seven) rows of second holes 143 at various distances from the center axis of the first plate 141 between the inner edge and the outer edge of the first plate 141. The second holes 143 of one column may be arranged close (e.g., at a first distance) to the inner edge of the first plate 141. The second holes 143 of one column may be arranged apart from the outer edge of the first plate 141 by a distance (e.g., a second distance that is greater than the first distance). The size of each of the plurality of second holes 143 may be smaller than the size of the first hole 142.

The second moving body 140 may include a plurality of second plates 144. For example, one of the plurality of second plates 144 may be disposed on a first side edge region of the first plate 141 and the other second plate may be disposed on a second side edge at an opposite side of the first side edge of the first plate 141. In an embodiment that is not shown, the second moving body 140 may include three or more second plates 144 disposed on different edges of the first plate 141, respectively. In an embodiment that is not shown, the second moving body 140 may include only a single second plate 144. Each end region of the plurality of second plates 144 may be connected to the side surface of the first plate 141. The plurality of second plates 144 may include a metal material. For example, the plurality of second plates 144 may include aluminum. The plurality of second plates 144 may be disposed to face a corresponding leg (e.g., the first leg 112, the second leg 113, or a third leg (not shown), etc.). For example, one second plate 144 may be disposed on the inner side (e.g., the +Y direction) of the first leg 112 and face the first leg 112, and the other second plate 144 may be disposed on the inner side (e.g., the -Y direction) of the second leg 113 and face the second leg 113. The plurality of second plates 144 may have a surface that is in a perpendicular direction (e.g., the X direction and/or the Y direction) that is different from the perpendicular direction (e.g., the Z direction) of the surface of the first plate 141. The plurality of second plates 144 may be parallel to the first leg 112 and/or the second leg 113.

The second moving body 140 may include a protrusion 145. The protrusion 145 may be configured to support the first moving body 130. Before conducting a drape test, a user may place the first moving body 130 on the protrusion 145. The protrusion 145 may be disposed at the center region of the first plate 141. The protrusion 145 may be configured to protrude from the inner edge of the first plate 141 towards the center axis (e.g., the Z axis) of the first plate 141. The protrusion 145 may be formed as a step on the inner edge of the first plate 141. The protrusion 145 may be formed along the edge of a lower region (e.g., an end region in the -Z axis direction) of the inner edge of the first plate 141. The protrusion 145 may be in the form of a step, but is not limited thereto. The protrusion 145 may be an arbitrary shape protruding towards the inner edge of the first plate 141.

The combined thickness of the thickness (e.g., thickness in the Z axis direction) of the protrusion 145 and the thickness (e.g., thickness in the Z axis direction) of the first moving body 130 may be substantially the same as the thickness (e.g., thickness in the Z axis direction) of the first plate 141. When the protrusion 145 supports the first plate 141, the height of a first surface (e.g., the surface in the +Z normal direction) of the first moving body 130 may be substantially the same as a height of a first surface (e.g., the surface in the +Z normal direction) of the first plate 141. The protrusion 145 may include a metal material. For example, the protrusion 145 may include aluminum.

The protrusion 145 may be disposed at any position in the first plate 141 that may support the first moving body 130. For example, in an embodiment that is not shown, the protrusion 145 may be disposed on a lower side (e.g., a side in the -Z normal direction) of the first plate 141 and may be configured to protrude towards the center axis of the first plate 141. The protrusion 145 may be configured to support the first moving body 130 on the lower side of the first plate 141. When the protrusion 145 is disposed on the lower side of the first plate 141, the thickness (e.g., the thickness in the Z axis direction) of the first moving body 130 may be substantially the same as the thickness (e.g., the thickness in the Z axis direction) of the first plate 141.

The fixing body 110, the first moving body 130, and the second moving body 140 may have a color having a lower saturation (e.g., black) than the color of the fabric (e.g., the fabric FB of FIGS. 5 to 7). This may increase the discernibility of the fabric for the fixing body 110, the first moving body 130, and the second moving body 140 by an optical element (e.g., the camera 190). For example, the fixing body 110, the first moving body 130, and the second moving body 140 may be anodized.

The apparatus 100 may include a fixing unit 150. The fixing unit 150 may be configured to fix the fabric (e.g., the fabric FB of FIGS. 5 to 7) to the extension 114. The fixing unit 150 may be configured to fix the first moving body 130 to the extension 114. The fixing unit 150 may include various structures (e.g., an electromagnet, an anchor, etc.) to fix the first moving body 130 and/or the fabric to the extension 114. For example, the fixing unit 150 may cause the first moving body 130 to be fixed directly and magnetically to the extension 114. For example, the fixing unit 150 may cause the first moving body 130 to be fixed to the extension 114 by magnetic coupling between other components (e.g., a first fixing element 151 and a second fixing element 152). The fixing unit 150 may reduce or prevent undesired shape changes of the fabric due to the air flow around the fabric while the first plate 141 falls. The fixing unit 150 may be configured to fix the first moving body 130 to the fixing body 110 or the extension 114 when the first moving body 130 moves to a predetermined height (e.g., a height that is substantially the same as the second height D2 of FIG. 6) from the base 111. When the first moving body 130 is fixed to the fixing body 110 or the extension 114, the second moving body 140 may move substantially in the direction of gravity (e.g., the -Z axis direction) relative to the first moving body 130.

The fixing unit 150 may include a first fixing element 151 disposed on the extension 114. The first fixing element 151 may include a magnet. The first fixing element 151 may be disposed on a bottom surface (e.g., a surface in the -Z direction) of the extension 114. The first fixing element 151 may be substantially disposed on the center axis (e.g., the Z axis) of the first plate 141. Although the drawing illustrates that the first fixing element 151 is disposed on the bottom surface of the extension 114, the example is not limited thereto, and the first fixing element 151 may be disposed at any position that may fix the first moving body 130 and/or the fabric.

The fixing unit 150 may include a second fixing element 152 disposed on the first moving body 130. The second fixing element 152 may include a magnet. The second fixing element 152 may be disposed on a bottom surface (e.g., a surface in the -Z direction) of the first moving body 130. The second fixing element 152 may include a magnet.

When the first moving body 130 moves to a predetermined height (e.g., the height that is substantially the same as the second height D2 of FIG. 6) from the base 111, the first fixing element 151 and the second fixing element 152 may be magnetically coupled to each other. The first moving body 130 and/or the fabric FB may be fixed between the first fixing element 151 and the second fixing element 152.

The fixing body 110 may include a plurality of linear guides 120. The plurality of linear guides 120 may be configured to guide linear movement of the second moving body 140. For example, the plurality of linear guides 120 may guide a substantial gravity direction fall of the second moving body 140. For example, the plurality of linear guides 120 may guide the movement of the second moving body 140 in a direction opposite to the substantial gravity direction.

The plurality of linear guides 120 may each include a rail 121 and a slider 122 configured to slide relative to the rail 121. One rail 121 may be disposed on the first leg 112 and the other rail 121 may be disposed on the second leg 113. The slider 122 may be disposed on one corresponding second plate 144.

The rail 121 may extend in an extending direction (e.g., the Z direction) of the first leg 112 or the second leg 113 along the inner surface of the first leg 112 or the second leg 113. The slider 122 may be disposed on a lower region of the outer surface of the second plate 144. In an embodiment that is not shown, the rail 121 may be disposed on the second plate 144 and the slider 122 may be disposed on the first leg 112 or the second leg 113.

The apparatus 100 may include an actuator 160 configured to move (e.g., elevate) the second moving body 140 in a direction (e.g., the +Z axis direction) that is substantially opposite to the direction of the gravity. The actuator 160 may be disposed on the first leg 112 or the second leg 113. The actuator 160 may be configured to slide the slider 122 relative to the rail 121 in a direction that is substantially opposite to the direction of the gravity. The actuator 160 may be configured to free-fall substantially in the direction of the gravity (e.g., the -Z axis direction) when the first plate 141 moves to a height that is substantially the same as a predetermined height (e.g., the second height D2 of FIG. 6). The actuator 160 may be configured to substantially not cause interference with the free fall of the second moving body 140 when the second moving body 140 falls. For example, while the actuator 160 elevates the second moving body 140, the actuator 160 may be coupled to the second plate 144 or the slider 122 and when the first plate 141 moves to a predetermined height (e.g., the second height D2 of FIG. 6), the coupling may be released.

The apparatus 100 may include a coupler 170. The coupler 170 may be configured to couple the second moving body 140 to the fixing body 110. The coupler 170 may be configured to control the falling of the second moving body 140. For example, in a coupled state, the second moving body 140 may not move relative to the fixing body 110 whereas in a decoupled state, the second moving body 140 may move relative to the fixing body 110. For example, the coupler 170 may be configured to couple the second moving body 140 to the fixing body 110 when the first plate 141 moves to a height that is substantially the same as a predetermined height (e.g., the second height D2 of FIG. 6) and when the coupler 170 decouples the fixing body 110 from the second moving body 140, the second moving body 140 may fall.

The coupler 170 may include a first electromagnetic element 171 and a second electromagnetic element 172 configured to electromagnetically couple each other. For example, the first electromagnetic element 171 and the second electromagnetic element 172 may each include an electromagnet. While current flows through each of the first electromagnetic element 171 and the second electromagnetic element 172, the first electromagnetic element 171 and the second electromagnetic element 172 may remain coupled to each other. On the other hand, when the current flowing through at least one of the first electromagnetic element 171 and the second electromagnetic element 172 is reduced or blocked, the first electromagnetic element 171 and the second electromagnetic element 172 may be separated from each other. The first electromagnetic element 171 may be disposed on an upper part of one leg (e.g., the first leg 112 or the second leg 113) and the second electromagnetic element 172 may be disposed on a lower part of one second plate 144 corresponding to the leg.

In an embodiment, the coupler 170 may include an electromagnetic element and a magnetic element. For example, the electromagnetic element may include an electromagnet and a magnet element may include a permanent magnet. While the current flows through the electromagnetic element, the electromagnetic element and the magnetic element may remain coupled to each other, whereas when the current flowing through the electromagnetic element is reduced or blocked, the electromagnetic element and the magnetic element may be separated from each other. For example, the electromagnetic element may be implemented as one of elements 171 and 172 illustrated in FIGS. 1 and 4 to 7, and the magnetic element may be implemented as the other of elements 171 and 172 illustrated in FIGS. 1 and 4 to 7.

The apparatus 100 may include a laser 180. The laser 180 may be configured to emits laser light towards the center axis (e.g., the Z axis) of the first plate 141 and/or the center axis (e.g., the Z axis) of the first moving body 130 when the first moving body 130 and/or the second moving body 140 are at a position (e.g., a position at a first height D1 in FIG. 5) before elevating. A user may place the fabric such that a center of the fabric (e.g., the fabric FB of FIGS. 5 to 7) coincides with the center of the first moving body 130 on which the laser light is incident.

The laser 180 may be positioned on the extension 114. The extension 114 may include a hole (not shown) through which the laser light passes such that the laser 180 emits the laser light onto the center axis of the first moving body 130. The fixing unit 150 may include a hole (not shown) through which the laser light passes such that the laser 180 may emit the laser light along the center axis of the first moving body 130.

In an embodiment that is not shown, the laser 180 may be located at any position from which the laser light is emitted towards the substantial center of the first moving body 130. In this case, the extension 114 or the fixing unit 150 may not include a hole through which the laser light passes. For example, although the drawing illustrates that the laser 180 is positioned on the center axis of the extension 114, the laser 180 may be placed at other positions on the extension 114. The laser 180 may be oriented to irradiate the laser light along the center axis of the first moving body 130 at an arbitrary position on the extension 114.

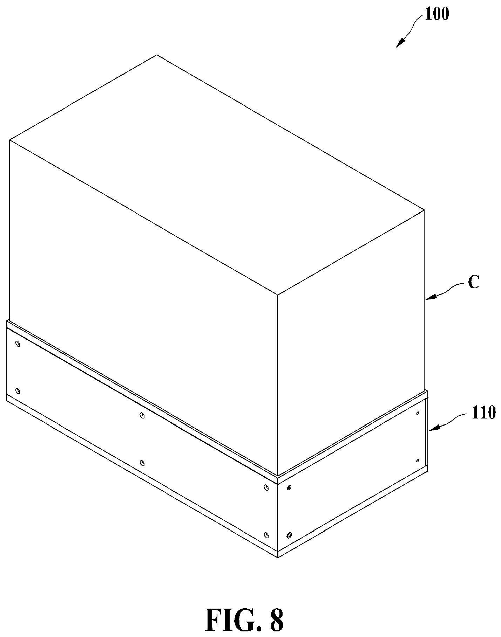

In an embodiment that is not shown, the laser 180 may be positioned on a component (e.g., a case C of FIG. 8) other than the extension 114. The laser 180 positioned on the other component may be oriented to irradiate the laser light along the center axis of the first moving body 130.

In an embodiment that is not shown, the apparatus 100 may not include the laser 180. Even when the apparatus 100 does not include the laser 180, the user may place the fabric (e.g., fabric FB of FIGS. 5 to 7) at a desired position on the first plate 141. For example, the fabric may include a mark (e.g., a "+" mark) that passes through the substantial center of the fabric, and when the user uses a portion where the mark extends and meets an edge portion of the fabric, the user may position the fabric such that the mark coincides with the substantial center of the first plate 141.

The apparatus 100 may include a camera 190. The camera 190 may be disposed on the base 111. The camera 190 may be disposed between the base 111 and the first moving body 130. The camera 190 may be disposed substantially on the center axis (e.g., the Z axis) of the first moving body 130. In an embodiment that is not shown, the camera 190 may be placed at other positions to obtain an image of the fabric. The camera 190 may be configured to obtain the image of the fabric by capturing a lower side (e.g., a side in the -Z direction) of the first moving body on the base 111. When the camera 190 is positioned below the first moving body, the apparatus 100 may be downsized compared to when the camera 190 is positioned above the first moving body. The camera 190 may be configured to have a viewing angle selected to obtain the image of the fabric from a relatively short distance (e.g., a distance of the second height D2 in FIG. 6). The viewing angle of the camera 190 may be configured to have an appropriate value according to a distance (e.g., the second height D2 in FIG. 6) between the elevated extension 114 and the camera 190.

Referring to FIG. 9, the apparatus 100 may include a texture scanner 192. The texture scanner 192 may be configured to scan the texture of the fabric (e.g., fabric FB of FIGS. 5 to 7). The texture scanner 192 may scan the texture by obtaining the image of the fabric. For example, when the first moving body 130 is positioned at a first height D1 from the base 111, the first plate 141 is also positioned at the first height D1 from the base, and the fabric FB is positioned on the first moving body and the first plate, the texture scanner 192 may scan the texture of the fabric. The texture scanner 192 may be positioned on the extension 114. The texture scanner 192 may be positioned on the extension 114. The texture scanner 192 may be positioned substantially on the center axis of the extension 114. Although the drawing does not illustrate the laser 180, both the laser 180 and the texture scanner 192 may be positioned on the extension 114. The apparatus 100 may include a light source L. The light source L may be disposed on the base 111. The light source L may be disposed on the base 111 and the camera 190 may be disposed on the light source L. The light source L may be configured to irradiate light towards the fabric (e.g., the fabric FB of FIGS. 5 to 7). The light source L may increase quality of the image capture by camera 190 to better discern the fabric. The light source L may include a light-emitting diode (LED). The light source L may enable the camera 190 to obtain an image of the fabric without shadow when the apparatus is enclosed in a case (e.g., the case C of FIG. 8) to conduct the drape test.

Unlike the case where the light source L is disposed on the base 111 and the camera 190 is disposed on the light source L, the light source L may be disposed on a region different from a region where the camera 190 is placed on the base 111. For example, the camera 190 may be disposed on a region (e.g., a center region of the base 111) on the base 111 and the light source L may be disposed on a region (e.g., an adjacent region of the base 111) which is different from the region and on which the camera 190 is not disposed. The light source L disposed on the adjacent region may at least partially enclose the camera 190.

The light source L may be configured to irradiate light towards the fabric (e.g., the fabric FB of FIGS. 5 to 7). The light source L may increase an ability of camera 190 to discern the fabric. The light source L may include an LED. The light source L may be configured to cause the camera 190 to obtain an image of the fabric without shadow in a case (e.g., the case C of FIG. 8) while conducting the drape test.

The apparatus 100 may include at least one second light source (not shown) disposed on the extension 114 or the guide 120. The second light source may improve the visibility of the texture when the texture scanner 192 scans the texture of the fabric (e.g., the fabric FB of FIGS. 5 to 7). The second light source may include an LED. The second light source may be configured to obtain an image of the fabric without shadow created by the texture scanner 192 or other components (e.g., the extension 114, etc.) while the texture scanner 192 scans the texture.

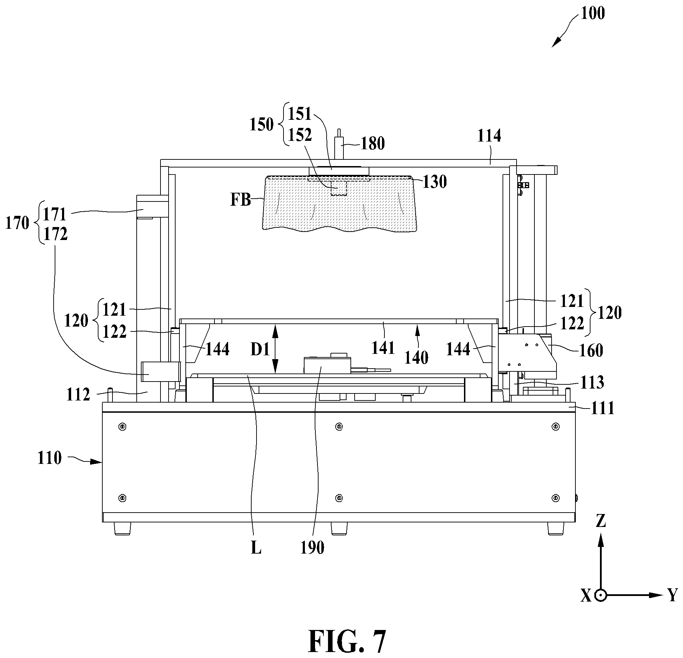

FIG. 5 is a side view of a drape testing apparatus before a second moving object elevates, according to an embodiment. FIG. 6 is a side view of a drape testing apparatus after a second moving object elevates, according to an embodiment. FIG. 7 is a perspective view of a drape testing apparatus after a second moving object descends according to an embodiment. FIG. 8 is a perspective view of a drape testing apparatus according to an embodiment.

Referring to FIGS. 5 to 8, a drape test process may be better understood. Referring to FIG. 5, to conduct a drape test, a user may place fabric FB on the first moving body (e.g., the first moving body 130 of FIG. 7) and the second moving body 140. The first moving body may be supported by the protrusion (e.g., the protrusion 145 of FIG. 3) and may be placed inside the first plate 141. The first moving body may be positioned at the first height D1 from the base 111 and the first plate 141 may also be positioned at the first height D1 from the base 111. The user may visually confirm a location on which the light emitted from the laser 180 incidents or a mark included in the fabric and may position the fabric such that the center of the fabric coincides with the center of the first moving body. The second fixing element 152 may be placed on a lower part of the first moving body. The second fixing element 152 may be substantially positioned on the center axis (e.g., the Z axis) of the first plate 141.

Referring to FIG. 6, the actuator 160 may move the second plate 144 in a direction (e.g., the +Z direction) that is substantially opposite to the direction of gravity to position the first moving body (e.g., the first moving body 130 of FIG. 7), the first plate 141, and the fabric FB at a second height D2 from the base 111. When the first moving body is positioned at the second height D2, the first moving body may be fixed to the extension 114. The first moving body may be fixed to the extension 114 as the first fixing element 151 and the second fixing element 152 are magnetically coupled to each other. When the first moving body is substantially positioned at the second height D2, the second fixing element 152 may move towards the first fixing element 151 by magnetism. When the second fixing element 152 moves towards the first fixing element 151, the first moving body 130 may also move. When the first plate 141 is positioned at the second height D2, the actuator 160 may no longer move the second plate 144. When the first moving body and the first plate 141 are positioned at the second height D2, the coupler 170 may couple the fixing body 110 to the second moving body 140. Even when the actuator 160 no longer moves the second plate 144, the height of the second moving body 140 may remain at the second height D2 by the coupler 170.

Referring to FIG. 7, while the first moving body 130 and the fabric FB are fixed to the extension 114, the second moving body 140 may move (e.g., fall) substantially in the direction of the gravity (e.g., the -Z direction) relative to the first moving body 130. The second moving body 140 may fall until the second moving body 140 is positioned at the first height D1 from the base 111. As a region of the fabric FB supported by the second moving body 140 extends, wrinkles may be formed in a region of the fabric FB that flows down. The camera 190 may obtain an image of the fabric FB. The second moving body 140 may move to the second height D2 where the first moving body 130 and the fabric FB are fixed. Thereafter, the first moving body 130 and the second moving body 140 may slowly return to the first height D1.

Referring to FIG. 8, the apparatus 100 may include a case C configured to block light from an outside. The case C may be configured to be detachable from the fixing body 110. The user may use the case C to reduce or remove noise due to background while conducting the drape test. The case C may have a color (e.g., black) with a lower saturation than the saturation of the fabric FB. The surface of the case C may include a paint of low saturation color. This may increase the discernibility of the fabric for the case C by an optical element (e.g., the camera 190). For example, the case C may be anodized. The user may conduct the drape test without the case C. Although FIGS. 5 to 7 illustrate a process of conducting the drape test without the case C, the user may perform the drape test process shown in FIGS. 5 to 7 while coupling the case C to the fixing body 110.

As described above, although the embodiments have been described with reference to the limited drawings, a person skilled in the art may apply various technical modifications and variations based thereon. For example, suitable results may be achieved if the described techniques are performed in a different order and/or if components in a described system, architecture, device, or circuit are combined in a different manner and/or replaced or supplemented by other components or their equivalents.

Accordingly, other implementations are within the scope of the following claims.

## Claims

1. A drape testing apparatus comprising:
a fixing body;
a first moving body configured to support a center region of a fabric, to move in a direction that is opposite to a direction of gravity, and to be fixed to the fixing body; and
a second moving body configured to support an adjacent region that is different from the center region of the fabric, to move in the direction that is opposite to the direction of gravity, and to move in the direction of gravity while the first moving body is fixed to the fixing body.

2. The drape testing apparatus of claim 1, wherein, while the first moving body is not fixed to the fixing body, the first moving body and the second moving body substantially simultaneously move in the direction opposite to the direction of gravity.

3. The drape testing apparatus of claim 1, wherein the second moving body comprises a hole aligned with the first moving body.

4. The drape testing apparatus of claim 1, wherein the second moving body comprises a protrusion configured to support the first moving body.

5. The drape testing apparatus of claim 1, further comprising:
a fixing unit configured to magnetically fix the first moving body to the fixing body.

6. The drape testing apparatus of claim 1, further comprising:
an actuator configured to move the second moving body in the direction opposite to the direction of gravity.

7. The drape testing apparatus of claim 1, further comprising:
a coupler configured to couple the second moving body to the fixing body.

8. The drape testing apparatus of claim 1, wherein the fixing body comprises:
a first leg;
a second leg opposite to the first leg based on the first moving body; and
an extension extending between the first leg and the second leg.

9. The drape testing apparatus of claim 1, further comprising:
a laser emitting a laser beam towards a center of the first moving body.

10. The drape testing apparatus of claim 1, further comprising:
a camera configured to obtain an image of the fabric and disposed below the first moving body.

11. The drape testing apparatus of claim 1, further comprising:
a texture scanner configured to scan a texture of the fabric.

12. The drape testing apparatus of claim 1, further comprising:
a light source at least partially illuminating the fabric.

13. The drape testing apparatus of claim 1, further comprising:
a case configured to block light from an outside and be detachable from the fixing body. alifteralong which the lifter slides
